# EUROPÄISCHE PATENTSCHRIFT

(11) **EP 3 128 942 B1**
(45) Veröffentlichungstag und Bekanntmachung des Hinweises auf die Patenterteilung: **07.03.2018**
(21) Anmeldenummer: 15745138.6
(22) Anmeldetag: 08.06.2015
(51) Int. Cl.: A61B 90/11, A61B 90/10, A61B 90/00, A61B 17/17

(54) **POSITIONIERHILFE FÜR CHIRURGISCHE EINGRIFFE**
POSITIONING AID FOR SURGICAL INTERVENTIONS
AUXILIAIRE DE POSITIONNEMENT POUR LES INTERVENTIONS CHIRURGICALES

(43) Veröffentlichungstag der Anmeldung: 15.02.2017
(73) Patentinhaber: HörSys IP GmbH & CO. KG, 30625 Hannover (DE)
(72) Erfinder: ORTMAIER, Tobias, 30966 Hemmingen (DE); MAJDANI, Omid, 30559 Hannover (DE); RAU, Thomas Stephan, 30853 Langenhagen (DE); LENARZ, Thomas, 30559 Hannover (DE); KOBLER, Jan-Philipp, 51766 Engelskirchen (DE); KLUGE, Marcel, 30451 Hannover (DE); JOHN, Samuel, 30519 Hannover (DE)
(74) Vertreter: Patentanwälte Thömen & Körner
(86) Internationale Anmeldenummer: PCT/DE2015/100227
(87) Internationale Veröffentlichungsnummer: WO 2016/198032

(56) Entgegenhaltungen:
- EP-A1- 3 058 890
- WO-A2-2008/014261
- CN-A- 101 766 505
- CN-A- 101 773 410
- US-A1- 2010 179 564
- US-A1- 2011 319 913
- Ramya Blachandran ET AL: "Minimally-Invasive, Image-Guided Cochlear Implantation for Pediatric Patients - Clinical Feasibility Study", Otolaryngol Head Neck Surg., 21. Januar 2014 (2014-01-21), Seiten 631-637, XP055246932, DOI: 10.1177/0194599813519050 Gefunden im Internet: URL:http://www.ncbi.nlm.nih.gov/pmc/articl es/PMC4431770/?report=reader [gefunden am 2016-02-02]
- ROBERT F. LABADIE ET AL: "Customized, rapid-production microstereotactic table for surgical targeting: description of concept and in vitro validation", INTERNATIONAL JOURNAL OF COMPUTER ASSISTED RADIOLOGY AND SURGERY, Bd. 4, Nr. 3, 1. Mai 2009 (2009-05-01), Seiten 273-280, XP055247211, DE ISSN: 1861-6410, DOI: 10.1007/s11548-009-0292-3

## Beschreibung

Die Erfindung betrifft eine Positionierhilfe für chirurgische Eingriffe nach dem Oberbegriff des Anspruchs 1.

Bei chirurgischen Eingriffen am Schädel, wie sie zum Beispiel für den Einsatz eines Cochlea-Implantats erforderlich sind, sah das konventionelle Vorgehen bisher das Freilegen aller relevanten Risikostrukturen der seitlichen Schädelbasis vor, um deren Unversehrtheit zu gewährleisten. Im Bereich zwischen Gesichts- und Geschmacksnerv wird anschließend das Mittelohr eröffnet, um darüber den Zugang zum Innenohr anzulegen.

Zur Reduzierung des Zeitaufwands, des operativen Risikos und des Traumas wird ein minimal-invasiver Ansatz verfolgt. Aufgrund präoperativer Bild- und Planungsdaten der individuellen Anatomie wird ein Zugang in Form einer einzelnen Stichkanalbohrung von der Schädeloberfläche zur Cochlea angelegt. Dabei wird auf das Freilegen der Risikostrukturen ebenso verzichtet, wie auf ein kontinuierliches Tracking von Patient und Instrumenten. Dies erfordert jedoch eine hochgenaue Umsetzung der Planungsdaten.

Aus der US 7981122 B2 ist bereits eine Positionierhilfe bekannt, die in Fachkreisen als Microtable bezeichnet wird. Die bekannte Positionierhilfe besteht aus einer Platte mit einer zentralen, senkrecht zur Oberfläche der Platte verlaufenden Bohrung und Beinen, die in nach Planungsdaten für den chirurgischen Eingriff gefrästen Taschen der Platte angebracht sind. Die Beine werden in abgestuften Längen vorgehalten und durch die Tiefe der Taschen wird bestimmt, wie weit die Beine aus der Platte vorstehen. Über die ausgewählten Längen der Beine und die Tiefen der Taschen kann die Platte am Schädelknochen räumlich positioniert und ausgerichtet werden. Über knochenverankerte Kugelmarker, die an den von der Platte entfernten Enden der Beine angeordnet sind, wird sie gleichzeitig befestigt.

Weitere Positionierhilfen mit den vorgenannten Merkmalen sind auch aus den Druckschriften ROBERT F. LABADIE ET AL: "Customized, rapid-production microstereotactic table for surgical targeting: description of concept and in vitro validation", INTERNATIONAL JOURNAL OF COMPUTER ASSISTED RADIOLOGY AND SURGERY, Bd. 4, Nr. 3, 1. Mai 2009 (2009-05-01), Seiten 273-280, XP055247211, DE ISSN: 1861-6410, D01: 10.1007/s11548-009-0292-3; US 2011/319913 A1; US 2010/179564 A1 und WO 2008/014261 A2 bekannt.

Zusätzlich zu einer Ausführung mit den vorgenannten Merkmalen ist in der Druckschrift Ramya Blachandran ET AL: "Minimally-Invasive, Image-Guided Cochlear Implantation for Pediatric Patients - Clinical Feasibility Study", Otolaryngol Head Neck Surg., 21. Januar 2014 (2014-01-21), Seiten 631-637, XP055246932, DOI: 10.1177/0194599813519050 ein selbsthaltender Retraktor beschrieben, der aus zwei um eine Schwenkachse schwenkbaren Schenkeln mit jeweils einem Spreizhaken an den Schenkeln besteht. Die Winkelstellung der Schenkel kann über eine Klemmvorrichtung fixiert werden. Über einem der schwenkbaren Schenkel sind zwei Referenzmarkierungen und über dem anderen Schenkel eine Referenzmarkierung jeweils in Form von Kugeln mittels Verlängerungen angebracht. Die Referenzmarkierungen sollen analog zu Knochenankern verwendet werden, wenn der Schädelknochen für die Aufnahme konventioneller Knochenankern zu dünn ist.

In der US 2011/319913 A1 ist eine Positionierhilfe für chirurgische Eingriffe beschrieben, die einen mittels Knochenankern befestigten Vorpositionierrahmen umfasst, auf dem ein als Gough-Steward-Platform ausgebildetes Aktuatorsystem angeordnet ist. Bei diesem Aktuatortorsystem handelt es sich um ein Hexapod mit zwei Plattformen, zwischen denen sechs motorisch gesteuerte, teleskopartig veränderbare Stützen angeordnet sind. Während eine Plattform fest mit dem Vorpositionierrahmen verbunden ist, kann die andere Plattform durch koordinierte Längenveränderung der Stützen in mehreren Axrichtungen verlagert werden und in Grenzen beliebige Positionen einnehmen. Die bewegliche Plattform soll dann Werkzeuge so positionieren können, dass deren Achse mit einer Trajektorie für einen Zugang zu dem Operationsfeld für den chirurgischen Eingriff übereinstimmt.

Aus den Druckschriften CN 101 773 410 A und CN 101 766 505 A sind Positionierhilfen bekannt, bei denen ein Werkzeug verschiebbar auf einer bogenförmigen Schiene angeordnet ist und die bogenförmige Schiene wiederum schwenkbar auf einer Platte angeordnet ist. Mittels dieser Positionierhilfen kann die Ausrichtung des Werkzeugs in zwei rechtwinklig zueinander stehenden Achsen verändert werden.

Die Befestigung der Beine in den Taschen der Platte erfordert eine individuelle Nachbearbeitung des Rohlings. Den knochenverankerten Kugelmarkern kommt eine Doppelfunktion zu. Sie fungieren als Registrierungsmarker und dienen zur Verankerung der Bohrschablone am Schädel. Das Design der knochenverankerten Kugelmarker ist daher ein Kompromiss aus beiden Anforderungen und führt zu einer eingeschränkten Genauigkeit.

Da die vorgegebene Achse der Bohrung in der Platte mit der Trajektorie für den Zugang zur Cochlea in Übereinstimmung gebracht werden muss, ist die Montage und Justierung der Positionierhilfe am Schädelknochen sehr zeit- und werkzeugaufwendig. Auch ist keine sterile Fertigung gewährleistet, sodass eine zusätzliche Sterilisation durchgeführt werden muss. Zusätzlich bietet diese Konstruktion nur eine eingeschränkte mechanische Festigkeit.

In der älteren EP 3 058 890 A1 ist eine Positionierhilfe beschrieben, umfassend ein Trägersystem und ein auf dem Trägersystem befestigbares Führungssystem. Das Führungssystem besteht aus einem Basismodul, einem Führungsteil und Verbindungsmitteln, die das Führungsteil mit dem Basismodul verbinden. Das Führungsteil, das später zur Aufnahme und Führung chirurgischer Elemente dient, wird individuell nach vorermittelten Koordinaten gegenüber dem Basismodul ausgerichtet und anschließend durch die Verbindungsmittel in Form einer aushärtbaren, ursprünglich plastisch verformbaren Masse am Basismodul fixiert.

Der Erfindung liegt die Aufgabe zugrunde, eine Positionierhilfe dahin gehend zu verbessern, dass sie belastbar, genau und schnell fertigbar, montier- und demontierbar ist.

Diese Aufgabe wird bei einer Positionierhilfe nach dem Oberbegriff des Anspruchs 1 durch die Merkmale dieses Anspruchs gelöst.

Weiterbildungen und vorteilhafte Ausgestaltungen ergeben sich aus den Unteransprüchen.

Die erfindungsgemäße Positionierhilfe ist zweiteilig ausgeführt und besteht aus einem Trägersystem und einer Schablone. Das Trägersystem ist wiederverwendbar und kann in mehreren Größen für unterschiedliche Schädelgrößen und - formen vorgehalten werden. Das Trägersystem umfasst eine Trägerplatte mit einer Aussparung. Unter einer Aussparung wird hier ein Hohlraum verstanden, der von einer zur anderen Flachseite der Trägerplatte führt und ganz oder teilweise umschlossen ist. Ein ganz umschlossener Hohlraum hat vorzugsweise in Draufsicht eine Kreisform, während ein teilweise umschlossener Hohlraum eine Einbuchtung darstellt, die z. B. ein u-förmiges, halbmond-förmiges, sichelförmiges Design aufweisen kann.

Die Schablone wird hingegen für jeden Eingriff speziell aus einem für das gewählte Trägersystem passenden Rohling endgefertigt. Dies kann ein einheitlicher, zunächst im Bereich möglicher Führungsöffnungen aus durchgehend vorhandenem Material bestehender Körper sein, in den die Führungsöffnung individuell nach vorermittelten Koordinaten durch Materialabtrag, wie z. B. Bohren, Fräsen eingebracht wird. Anhand von zuvor erstellten Planungsdaten für den chirurgischen Eingriff werden Koordinaten einer Trajektorie für einen Zugang zu dem Operationsfeld für den chirurgischen Eingriff errechnet. Durch Transformation dieser Koordinaten auf die Schablone werden der Ort und die Ausrichtung für die Führungsöffnung in der Schablonenplatte festgelegt und die Führungsöffnung als Bohrung oder Fräsung ausgeführt. Die Schablonenplatte mit der Führungsöffnung ist ein nur einmalig eingesetztes Bauteil, da Ort und Ausrichtung der Führungsöffnung einen Bezug zur individuellen Anatomie eines Patienten und dem Befestigungsort des Trägersystems am Schädelknochen haben.

Somit kann zunächst das Trägersystem in Grenzen frei am Schädelknochen positioniert werden ohne auf die Trajektorie zur Schaffung des Zugangs zum Operationsfeld achten zu müssen. Dadurch kann die Trägerplatte des Trägersystems dicht am Schädelknochen angebracht werden. Sie wird dabei vorzugsweise parallel zu einer Tangentialebene des Schädelknochens ausgerichtet, deren Berührungspunkt etwa im Zentrum der Trägerplatte liegt. Die sich daraus ergebenden kurzen Abstände der Ränder der Trägerplatte zum Schädelknochen ermöglichen ebenso kurze Stützen, was sich vorteilhaft auf deren mechanische Stabilität auswirkt. Denn die Auslenkung der Stützen ist bei Krafteinwirkung geringer, wodurch eine höhere Genauigkeit der Ausrichtung der chirurgischen Werkzeuge gewährleistet wird.

Da das Trägersystem vorgefertigt ist, kann es im sterilen Zustand vorgehalten werden. Bei der Schablone ist lediglich die Führungsöffnung in die Schablonenplatte einzubringen, was unter sterilen Bedingungen erfolgen kann. Die Zeit zwischen dem Beginn der Montage der Positionierhilfe bis zum Beginn des chirurgischen Eingriffs kann gegenüber anderen Systemen deutlich verringert werden.

Für die der Trägerplatte abgewandten Enden der Stützen sind mehrere Varianten vorgesehen. Die Enden können entweder
a) ein Lochraster aufweisen, wobei die Löcher Durchgriff- und Befestigungsorte von Knochenankern sind oder
b) eine Vielzahl von Knochenankern umfassen oder
c) dornenartige Stifte tragen, die mittels Knochenschrauben zum Schädelknochen verspannbar sind.

Durch diese möglichen Arten der Befestigung lässt sich das Trägersystem so stabil am Schädelknochen verankern, dass die erforderliche Maßhaltigkeit für den Einsatz der chirurgischen Werkzeuge gewährleistet ist und auch die während des Eingriffs auftretenden Kräfte ohne Lageänderung der Positionierhilfe aufgefangen werden.

Es kann vorgesehen sein, dass die Stützen Sollbruchstellen umfassen.

Dadurch ist es im Notfall möglich, das Trägersystem auch ohne Lösen der Verankerungen im Schädelknochen schnell zu entfernen.

Für eine genaue Ausrichtung der Schablonenplatte auf der Trägerplatte können alternative Mittel vorgesehen sein. So besteht die Möglichkeit, dass
a) auf einer der beiden Platten, nämlich der Trägerplatte oder der Schablonenplatte, Stifte und auf der jeweils anderen Platte, nämlich der Schablonenplatte oder der Trägerplatte Ausnehmungen zur Aufnahme der Stifte angeordnet sind und im Montagezustand die Stifte der einen Platte in den Ausnehmungen der anderen Platte stecken oder
b) auf beiden Platten, nämlich sowohl der Trägerplatte als auch der Schablonenplatte, Ausnehmungen zur Aufnahme von Stiften vorhanden sind, und im Montagezustand Stifte jeweils in beiden Ausnehmungen der Platten stecken, und dass die Platten gegeneinander verspannt sind.

Dies kann z. B. in an sich bekannter Weise mittels Schrauben, Federn oder Klammern realisiert sein

Beide Varianten ermöglichen eine spielfreie Verbindung zwischen der Schablonenplatte und der Trägerplatte.

Die Stifte oder Ausnehmungen können jeweils mindestens zweifach vorhanden und unterschiedlich ausgeführt und/oder asymmetrisch angeordnet sein.

Bereits zwei Stifte oder Ausnehmungen sind bei geschickter Verteilung über das Trägersystem eindeutig. Wenn sie zusätzlich noch unterschiedlich ausgeführt sind, z. B. unterschiedliche Durchmesser oder Querschnittsformen aufweisen, passen sie nur noch in einer möglichen Ausrichtung zusammen. Alternativ sind drei asymmetrisch angeordnete Stifte oder Ausnehmungen geeignet.

Dadurch wird erreicht, dass sich die Schablonenplatte mit der Trägerplatte nur in einer einzigen möglichen Ausrichtung zueinander verbinden lässt. Irrtümliche Fehlausrichtungen werden so sicher vermieden.

Vorzugsweise ist in der Führungsöffnung ein austauschbarer zylindrischer Werkzeugführungskörper angeordnet.

Der Werkzeugführungskörper kann so speziell für den Einsatz chirurgischer Werkzeuge bemessen sein und entfernt werden, um Platz für die Einbringung eines Implantats zu gewähren.

Vorzugsweise weist der Werkzeugführungskörper einen Anschlag für die Tiefeneinstellung eines Werkzeugs auf.

Damit kann automatisch die Eindringtiefe eines chirurgischen Werkzeugs begrenzt werden.

Weiterhin kann der Werkzeugführungskörper eine Drehsicherung in Form eines Stiftes aufweisen, der in eine neben der Führungsöffnung in der Schablonenplatte angeordnete Ausnehmung eingreift.

Dadurch wird verhindert, dass der Werkzeugführungskörper bei Ausübung eines Drehmoments mitgedreht wird.

Die Aussparung in der Trägerplatte und die Führungsöffnung in der Schablonenplatte können einen nach außen führenden Schlitz oder eine U-förmige offene Gestalt aufweisen.

Dadurch ist es möglich, bei Einsatz eines Implantats Kabel von der Seite her der Positionierhilfe zuzuführen.

Weiterhin können am Trägersystem Registrierungsmarkierungen angeordnet sein oder das Trägersystem selbst oder Teile davon können selbst als Registrierungsmarkierungen ausgeführt sein.

Die Registrierungsmarkierungen ermöglichen die räumliche Erfassung des am Schädelknochen angebrachten Trägersystems relativ zur Anatomie des Schädels des Patienten im Rahmen einer dreidimensionalen Bildgebung. Die genaue räumliche Erfassung des am Schädelknochen angebrachten Trägersystems wiederum ist Voraussetzung für die Übertragung der Koordinaten der Führungsöffnung in der Schablonenplatte, damit deren Achse nach der Montage der Schablonenplatte auf der Trägerplatte mit der Trajektorie für den Zugang zum Operationsfeld übereinstimmt.

Nachfolgend wird die Erfindung anhand eines Ausführungsbeispiels erläutert, das in der Zeichnung dargestellt ist. Darin zeigen:
- Fig. 1: eine perspektivische Ansicht einer Positionierhilfe in Explosionsdarstellung und
- Fig. 2: eine perspektivische Ansicht der Positionierhilfe im montierten Zustand.

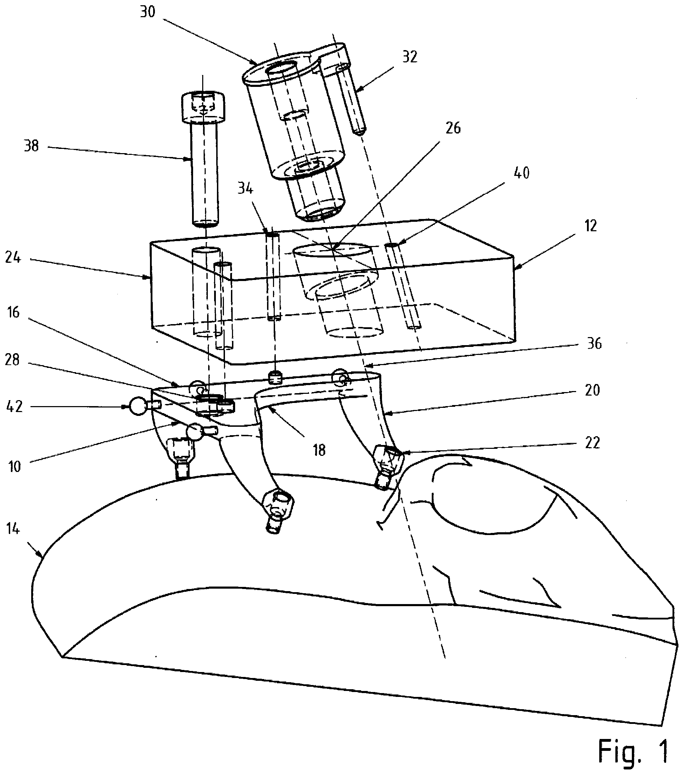

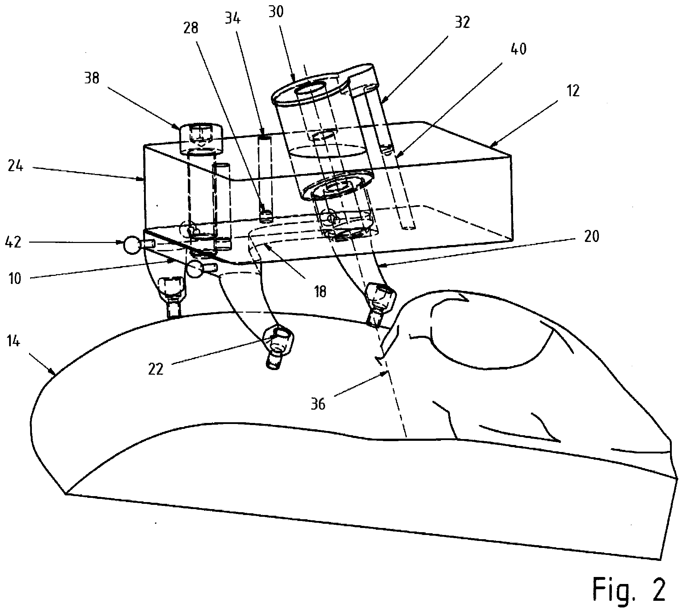

Die in den Figuren 1 und 2 gezeichnete Positionierhilfe für chirurgische Eingriffe umfasst ein Trägersystem 10 und eine Schablone 12. Das Trägersystem 10 besteht aus einer mit einer Aussparung 18 versehenen Trägerplatte 16 und Stützen 20, mittels denen die Trägerplatte 16 über einem Operationsfeld für den chirurgischen Eingriff an einem Schädelknochen 14 befestigt ist. Die Schablone 12 besteht aus einer mit der Trägerplatte 16 spielfrei und formschlüssig verbindbaren Schablonenplatte 24 mit einer Führungsöffnung. Die Längsmittelachse 36 dieser Führungsöffnung 26 ist individuell nach vorermittelten Koordinaten auf einen Zugang zu dem Operationsfeld für den chirurgischen Eingriff angeordnet und ausgerichtet sein. Sie ist, wie im Ausführungsbeispiel dargestellt, exzentrisch zur Schablonenplatte 24 angeordnet und weist eine von der Senkrechten der Oberfläche der Schablonenplatte 24 abweichende Neigung ihrer Längsmittelachse 36 auf.

Die Aussparung 18 in der Trägerplatte 16 ist so groß bemessen, dass die Führungsöffnung 26 in der Schablonenplatte 24 auch bei maximaler exzentrischer Anordnung nicht von der Trägerplatte 16 abgedeckt wird. An drei Orten der dem Schädelknochen 14 zugewandten Flachseite der Trägerplatte 16 befinden sich Stützen 20, mit denen die Trägerplatte 16 am Schädelknochen 14 abgestützt ist und fixiert werden kann. An den Enden befinden sich Füße 22 mit dornenartigen Stiften. Die Füße 22 stützen sich auf der Haut oder dem Schädelknochen 14 ab. Die Trägerplatte 16 ist durch eine Knochenschraube mit dem Schädelknochen verspannt.

Nahe den Kanten der Trägerplatte 16 befinden sich Stifte 28, die zur Schablonenplatten 24 vorstehen und mit Ausnehmungen 34 der Schablonenplatte 24 fluchten. Die Stifte 28 und Ausnehmungen 34 sind asymmetrisch angeordnet und gestatten ein Zusammensetzen von Schablonenplatte 24 und Trägerplatte 16 nur in einer einzigen von drei sich zunächst anbietenden Winkelpositionen. Die Schablonenplatte 24 wird hier mit der Trägerplatte 16 durch drei Schrauben 38 fixiert, die auf Gewinde der Stifte 28 aufgeschraubt werden. Durch die Stifte 28 und Ausnehmungen 34 sind die Schablonenplatte 24 und die Trägerplatte 16 zueinander spielfrei ausgerichtet.

Oberhalb der Führungsöffnung 26 in der Schablonenplatte 24 befindet sich ein Werkzeugführungskörper 30, der zur Führung und Halterung eines chirurgischen Werkzeugs dient. Am Werkzeugführungskörper 30 ist eine Drehsicherung in Form eines Stiftes 32 angeordnet, der beim Einsetzen des Werkzeugführungskörpers 30 in die Führungsöffnung 26 in eine Ausnehmung 40 der Schablonenplatte 24 eingreift.

Während in Fig. 1 die Positionierhilfe als Explosionsdarstellung gezeichnet ist, sind die einzelnen Bestandteile in Fig. 2 im montierten Zustand dargestellt.

Bei der Vorbereitung eines chirurgischen Eingriffs wird zunächst die Anatomie des Schädels eines Patienten mittels bildgebender Verfahren erfasst und daraufhin der chirurgische Eingriff geplant. Ein Bestandteil dieser Planung ist die Ausrichtung einer gemeinsamen Achse für eine Bohrung oder Fräsung des Schädelknochens und den weiteren Zugang zum Operationsfeld. Anhand der Größe und Form des Schädels wird ein Trägersystem 10 ausgewählt, das auf dem Schädelknochen 14 fixiert wird. Um die räumliche Zuordnung des am Schädelknochen 14 befestigten Trägersystems 10 relativ zur Anatomie des Schädels des Patienten zu bestimmen, dienen die am Trägersystem 10 angebrachten Registrierungsmarkierungen 42, die einen möglichst großen gegenseitigen Abstand aufweisen. Diese Registrierungsmarkierungen 42 werden im Rahmen einer anschließenden dreidimensionalen Bildgebung des Patienten deutlicher als die übrigen Bestandteile des Trägersystems 10 dargestellt und erleichtern so die Erfassung der Koordinaten des Trägersystems 10. Die Koordinaten des Trägersystems 10 sowie der Trajektorie für den Zugang zum Operationsfeld werden auf die Schablonenplatte 24 übertragen. Daraus werden der Ort und die Neigung für die Führungsöffnung 36 in der Schablonenplatte 24 bestimmt und diese gefertigt. Danach wird die Schablonenplatte 24 auf der Trägerplatte 16 fixiert und der Werkzeugführungskörper 30 eingesetzt.

Damit sind die mechanischen Voraussetzungen für eine genaue Führung des chirurgischen Werkzeugs zur Öffnung des Schädelknochens, Vordringen zum Operationsfeld und Einsetzen des Implantats abgeschlossen.

### Bezugszeichenliste

- 10: Trägersystem
- 12: Schablone
- 14: Schädelknochen
- 16: Trägerplatte
- 18: Aussparung
- 20: Stützen
- 22: Füße
- 24: Schablonenplatte
- 26: Führungsöffnung
- 28: Stifte
- 30: Werkzeugführungskörper
- 32: Stift
- 34: Ausnehmungen
- 36: Längsmittelachse
- 38: Schrauben
- 40: Ausnehmung
- 42: Registrierungsmarkierungen

## Patentansprüche

1. Positionierhilfe für chirurgische Eingriffe, umfassend ein Trägersystem (10) und eine Schablone (12), wobei das Trägersystem (10) aus einer mit einer Aussparung (18) versehenen Trägerplatte (16) und Stützen (20), mittels denen die Trägerplatte (16) über einem Operationsfeld für den chirurgischen Eingriff an einem Schädelknochen (14) befestigbar ist, besteht, und die Schablone (12) aus einer mit der Trägerplatte (16) spielfrei verbindbaren Schablonenplatte (24) mit einer Führungsöffnung (26) besteht, wobei die Schablonenplatte (24) aus einem Rohling besteht, in dem die Führungsöffnung (26) durch Materialabtrag des Rohlings individuell nach vorermittelten Koordinaten angeordnet und ausgerichtet ist, und die Längsmittelachse (36) der Führungsöffnung (26) im zusammengesetzten Zustand von Trägersystem (10) und Schablone (12) und am Schädelknochen (14) befestigtem Trägersystem (10) mit einer Trajektorie für einen Zugang zu dem Operationsfeld für den chirurgischen Eingriff übereinstimmt, **dadurch gekennzeichnet, dass** die Längsmittelachse (36) der Führungsöffnung (26) exzentrisch zur Schablonenplatte (24) angeordnet ist, eine von der Senkrechten der Oberfläche der Schablonenplatte (24) abweichende Neigung ihrer Längsmittelachse (36) aufweist, und die Aussparung in der Trägerplatte (16) so groß bemessen ist, dass die Führungsöffnung (26) in der Schablonenplatte (24) auch bei maximaler exzentrischer Anordnung nicht von der Trägerplatte (16) abgedeckt wird.

2. Positionierhilfe nach Anspruch 1, **dadurch gekennzeichnet, dass** die Stützen (20) an ihren der Trägerplatte abgewandten Enden entweder
a) ein Lochraster aufweisen, wobei die Löcher Durchgriff- und Befestigungsorte von Knochenankern sind oder
b) eine Vielzahl von Knochenankern umfassen oder
c) dornenartige Stifte tragen, die mittels Knochenschrauben zum Schädelknochen verspannbar sind.

3. Positionierhilfe nach Anspruch 1 oder 2, **dadurch gekennzeichnet, dass** die Stützen Sollbruchstellen umfassen.

4. Positionierhilfe nach einem der Ansprüche 1 bis 3, **dadurch gekennzeichnet, dass**
a) auf einer der beiden Platten, nämlich der Trägerplatte (16) oder der Schablonenplatte (24), Stifte (28) und auf der jeweils anderen Platte, nämlich der Schablonenplatte (24) oder der Trägerplatte (16) Ausnehmungen (34) zur Aufnahme der Stifte (28) angeordnet sind und im Montagezustand die Stifte (28) der einen Platte in den Ausnehmungen (34) der anderen Platte stecken oder
b) auf beiden Platten, nämlich sowohl der Trägerplatte (16) als auch der Schablonenplatte (24), Ausnehmungen zur Aufnahme von Stiften vorhanden sind, und im Montagezustand Stifte jeweils in beiden Ausnehmungen der Platten stecken, und dass die Platten gegeneinander verspannt sind.

5. Positionierhilfe nach Anspruch 4, **dadurch gekennzeichnet, dass** die Stifte (28) oder Ausnehmungen (34) jeweils mindestens zweifach vorhanden und unterschiedlich ausgeführt und/oder asymmetrisch angeordnet sind.

6. Positionierhilfe nach einem der Ansprüche 1 bis 5, **dadurch gekennzeichnet, dass** in der Führungsöffnung (26) ein austauschbarer zylindrischer Werkzeugführungskörper (30) angeordnet ist.

7. Positionierhilfe nach Anspruch 6, **dadurch gekennzeichnet, dass** der Werkzeugführungskörper (30) einen Anschlag für die Tiefeneinstellung eines Werkzeugs aufweist.

8. Positionierhilfe nach Anspruch 6 oder 7, **dadurch gekennzeichnet, dass** der Werkzeugführungskörper (30) eine Drehsicherung aufweist, der in eine neben der Führungsöffnung (26) in der Schablonenplatte (24) angeordnete Ausnehmung (40) eingreift.

9. Positionierhilfe nach einem der Ansprüche 1 bis 8, **dadurch gekennzeichnet, dass** die Aussparung (18) in der Trägerplatte (16) und die Führungsöffnung (26) in der Schablonenplatte (24) einen nach außen führenden Schlitz oder eine U-förmige offene Gestalt aufweisen.

10. Positionierhilfe nach einem der Ansprüche 1 bis 9, **dadurch gekennzeichnet, dass** am Trägersystem (10) Registrierungsmarkierungen (42) angeordnet sind oder das Trägersystem selbst oder Teile davon als Registrierungsmarkierungen ausgeführt sind.

## Claims

1. A positioning aid for surgical interventions, comprising a carrier system (10) and a template (12), wherein the carrier system (10) consists of a carrier plate (16) provided with a recess (18), and of supports (20), by means of which the carrier plate (16), over an operating field for surgical intervention, can be fixed to a cranial bone (14), and the template (12) consists of a template plate (24) which can be connected in a play-free manner to the carrier plate (16), said template plate (24) having a guiding aperture (26), wherein the template plate (24) consists of a blank in which the guiding aperture (26) is arranged and oriented individually in accordance with predetermined coordinates by means of removal of material of the blank,
and the longitudinal central axis (36) of the guiding aperture (26), when the carrier system (10) and template (12) are in the assembled state and the carrier system (10) is fixed to the cranial bone (14), corresponds to a trajectory for an access to the operating field for the surgical intervention, **characterised in that** the longitudinal central axis (36) of the guiding aperture (26) is arranged eccentrically relative to the template plate (24), has an incline of the longitudinal central axis (36) thereof which deviates from the vertical of the surface of the template plate (24), and the recess in the carrier plate (16) is dimensioned to be of such a size that the guiding aperture (26) in the template plate (24) is not covered by the carrier plate (16) even in the case of maximum eccentric arrangement.

2. The positioning aid according to Claim 1, **characterised in that** the supports (20), at the ends thereof facing away from the carrier plate, either
a) have a hole pattern, wherein the holes are penetration and fixing locations of bone anchors or
b) comprise a plurality of bone anchors or
c) bear mandrel-type pins which can be tightened to the cranial bone by means of bone screws.

3. The positioning aid according to Claim 1 or 2, **characterised in that** the supports comprise predetermined breaking points.

4. The positioning aid according to any one of Claims 1 to 3, **characterised in that**
a) pins (28) are arranged on one of the two plates, namely the carrier plate (16) or the template plate (24), and recesses (34) for receiving the pins (28) are arranged on the respective other plate, namely the template plate (24) or the carrier plate (16), and in the mounted state the pins (28) of one plate fit into the recesses (34) of the other plate, or
b) on both plates, namely both the carrier plate (16) and the template plate (24), there are recesses present for receiving pins, and in the mounting state pins respectively fit into both recesses of the plates and
**in that** the plates are tightened against one another.

5. The positioning aid according to Claim 4, **characterised in that** at least two pins (28) or recesses (34) are respectively present and are designed differently and/or are arranged asymmetrically.

6. The positioning aid according to any one of Claims 1 to 5, **characterised in that** an exchangeable cylindrical tool-guiding body (30) is arranged in the guiding aperture (26).

7. The positioning aid according to Claim 6, **characterised in that** the tool-guiding body (30) has a stopper for the depth adjustment of a tool.

8. The positioning aid according to Claim 6 or 7, **characterised in that** the tool-guiding body (30) has an anti-rotation device which engages in a recess (40) arranged beside the guiding aperture (26) in the template plate (24).

9. The positioning aid according to any one of Claims 1 to 8, **characterised in that** the recess (18) in the carrier plate (16) and the guiding aperture (26) in the template plate (24) have an outwardly leading slot or a U-shaped open form.

10. The positioning aid according to any one of Claims 1 to 9, **characterised in that** registering markings (42) are arranged on the carrier system (10) or the carrier system itself or parts thereof are designed as registering markings.

## Revendications

1. Auxiliaire de positionnement destiné à des interventions chirurgicales, l'auxiliaire de positionnement comprenant un système de support (10) et un gabarit (12), le système de support (10) étant composé d'une plaque de support (16) pourvue d'un évidement (18) et de supports (20), au moyen desquels la plaque de support (16) peut être fixée à un os crânien (14), au-dessus d'un champ opératoire destiné à l'intervention chirurgicale, et le gabarit (12) étant composé d'une plaque de gabarit (24) pouvant être reliée sans jeu à la plaque de support (16) et comportant une ouverture de guidage (26), la plaque de gabarit (24) étant une ébauche, dans laquelle l'ouverture de guidage (26) est disposée et orientée individuellement par enlèvement de matière de l'ébauche selon des coordonnées déterminées à l'avance et, en état assemblé du système de support (10) et du gabarit (12) et du système de support (10) fixé à l'os crânien (14), l'axe central longitudinal (36) de l'ouverture de guidage (26) concorde avec une trajectoire donnant accès au champ opératoire destiné à l'intervention chirurgicale, **caractérisé en ce que** l'axe central longitudinal (36) de l'ouverture de guidage (26) est disposé de manière excentrique par rapport à la plaque de gabarit (24), qu'il présente une inclinaison s'écartant de la verticale de la surface de la plaque de gabarit (24) et que l'évidement pratiqué dans la plaque de support (16) est dimensionné tel que l'ouverture de guidage (26) dans la plaque de gabarit (24) ne soit pas recouverte par la plaque de support (16) même à disposition excentrique maximale.

2. Auxiliaire de positionnement suivant la revendication 1, **caractérisé en ce que** les supports (20)
a) présentent à leurs extrémités opposées à la plaque de support une trame perforée, les perçages étant des endroits de passage et de fixation d'ancres osseux, ou
b) comprennent une pluralité d'ancres osseux, ou
c) portent des tiges en forme de griffe pouvant être contraintes par rapport à l'os crânien au moyen de vis à os.

3. Auxiliaire de positionnement suivant la revendication 1 ou 2, **caractérisé en ce que** les supports comprennent des points destinés à la rupture.

4. Auxiliaire de positionnement suivant une des revendications 1 à 3, **caractérisé en ce que**
a) des tenons (28) sont disposés sur une des deux plaques, à savoir sur la plaque de support (16) ou la plaque de gabarit (24) et des évidements (34) destinés à recevoir les tenons (28) sont disposés sur l'autre plaque, à savoir la plaque de gabarit (24) ou la plaque de support (16), et **en ce qu'**en état monté, les tenons (28) de l'une des plaques sont insérés dans les évidements (34) de l'autre plaque, ou
b) des évidements destinés à recevoir des tenons existent dans les deux plaques, à savoir non seulement dans la plaque de support (16), mais encore dans la plaque de gabarit (24), et qu'en état monté, des tenons sont insérés respectivement dans les deux évidements des plaques et que les plaques sont contraintes l'une par rapport à l'autre.

5. Auxiliaire de positionnement suivant la revendication 4, **caractérisé en ce que** les tenons (28) ou les évidements (34) existent respectivement en double et sont de conception différente et/ou disposés de manière asymétrique.

6. Auxiliaire de positionnement suivant une des revendications 1 à 5, **caractérisé en ce qu'**un corps cylindrique de guidage d'outil (30) échangeable est disposé dans l'ouverture de guidage (26).

7. Auxiliaire de positionnement suivant la revendication 6, **caractérisé en ce que** le corps cylindrique de guidage d'outil (30) présente une butée pour le réglage en profondeur d'un outil.

8. Auxiliaire de positionnement suivant la revendication 6 ou 7, **caractérisé en ce que** le corps cylindrique de guidage d'outil (30) présente un dispositif anti-rotation qui prend dans un évidement (40) pratiqué dans la plaque de gabarit (24), à côté de l'ouverture de guidage (26).

9. Auxiliaire de positionnement suivant une des revendications 1 à 8, **caractérisé en ce que** l'évidement (18) dans la plaque de support (16) et l'ouverture de guidage (26) dans la plaque de gabarit (24) présentent une fente débouchant à l'extérieur ou une forme en U ouverte.

10. Auxiliaire de positionnement suivant une des revendications 1 à 9, **caractérisé en ce que** des marques de repérage (42) sont disposées sur le système de support (10) ou le système de support lui-même ou des parties de celui-ci sont conçus comme marques de repérage.
